# EUROPEAN PATENT APPLICATION

(11) **EP 2 388 680 A1**
(43) Date of publication of application: **23.11.2011**
(21) Application number: 10163031.7
(22) Date of filing: 17.05.2010
(51) Int. Cl.: G06F 3/01, A61B 5/0478

(54) **Apparatus and method for controlling at least one device, a corresponding computer program and a corresponding computer-readable storage medium**

(71) Applicant: Otto-von-Guericke-Universität Magdeburg, 39120 Magdeburg (DE)
(72) Inventor: Düzel, Emrah, 14467, Potsdam (DE)
(74) Representative: Gulde Hengelhaupt Ziebig & Schneider

(57) **Abstract**

The invention relates to an apparatus and to a method for controlling at least one device as well as to a corresponding computer program and a corresponding computer-readable storage medium, with the invention providing bio-feedback or a brain computer interface (BCl) for controlling for example mobile devices like mobile phones, personal digital assistants (PDA), media player or such, using the same hardware for hearing music as well as for controlling the mobile devices.

For controlling at least one device an apparatus is proposed, where the apparatus comprises at least one recording electrode (610) which is arrangeable in the outer ear canal (110), and one data processing unit. The apparatus is configured in such a way that signals of an electroencephalogram (EEG) are detectable by the at least one recording electrode (610), brain activities are decoded from the signals of the EEG and the at least one device is controlled based on results of the decoding.

## Description

The invention relates to an apparatus and to a method for controlling at least one device as well as to a corresponding computer program and a corresponding computer-readable storage medium, with the invention providing bio-feedback or a brain computer interface (BCI) for controlling for example mobile devices like mobile phones, personal digital assistants (PDA), media player or such, using the same hardware for hearing music as well as for controlling the mobile devices.

In the field of the invention some solutions are proposed. For example, the publication JP 02026533 A proposes a feedback device which is equipped with an electrode part for fetching the electric signal similar to the brain wave from the ear.

Another physiological condition sensing device is disclosed in JP 04208135 A. The device comprises an earphone, which is equipped with a pair of electrodes, which are connected to an α-wave sensor. The α-wave sensor is fitted with an amplifier, band-pass filter, comparator and tone signal generator. The tone signal generator is connected with a sound emitting element of the earphone. According to this solution the physiological condition sensor is located in that part of the earphone which contacts the skin.

A biological information measuring device is described in JP 2006-102163 A. The biological information measuring device has a rodlike biological information sensor inserted into the external auditory meatus of one of the external ears of a subject. The sensor measures biological information of the subject. Further, the device has a clamping portion for clamping the part of the other external ear, and a head band comprising a bowlike elastomer measuring connecting the living body measuring sensor and the clamping portion.

In JP 2008-067911 A an ear canal electrode unit and a biological information measuring apparatus is described. The electrode unit has an ear canal contact member made of a conductive material that contacts the ear canal partially with intervals. The ear canal contact member further has a communication hole for transmitting sounds to the eardrum.

A method and an apparatus for quantitatively evaluating mental states based on brain wave signal processing system is proposed in the international patent application WO 2008/091323 A1. There is a noise-free portable electroencephalogram (EEG) is provided. The system has hardware and software and can evaluate mental state quantitatively. The
quantitative data of mental states and their levels can be applied to brain machine interfaces like consumer products, video games, toys, military and aerospace as biofeedback or neurofeedback. The EEG signal is collected from the forehead.

In WO 2009/102430 A1 an audio headset with bio-signal sensors is disclosed. The headset comprises a first side portion having a first earpiece attached so that the first earpiece can be placed on top of, over, or inside a user's ear. The audio headset may include one or more EEG sensors positioned inside the first earpiece that is capable of resting against the skin of the ear when the audio headset is worn. It is not disclosed, whether the sensors positioned inside the first earpiece is used as signal sensor or as reference sensor.

In the publication Saddique, Syed M., Laraib Hassan Siddiqui: EEG based Brain Computer Interface, Journal of Software, Vol. 4, No. 6, August 2009, use of brain signals via scalp EEG is described to get control of a robot's navigation.

The prior art solutions using ear canal electrodes, however, provide only biological information, and are therefore not or only with restriction usable as BCI. Use of scalp and forehead electrodes, on the other hand, is cumbersome and involve some cosmetic problems.

It is therefore an object of the present invention to provide an apparatus and a method for controlling at least one device and a corresponding computer program and a corresponding computer-readable storage medium, which obviate the disadvantages of the conventional solutions and more particularly enable easy controlling of mobile devices like mobile phones, PDAs, media players or such.

This object is attained according to the invention with the features recited in claims 1, 9, 14 and 15. Advantageous embodiments of the invention are recited in the dependent claims.

According to a particular advantage of the invention, in a first embodiment of the inventive apparatus for controlling mobile devices no additional hardware is necessary. This is achieved by providing an apparatus which comprises at least one recording electrode, where the recording electrode is arrangeable in the outer ear canal. With the recording electrode EEG signals are recorded at the outer ear canal. In a preferred embodiment the recording electrode is positioned at the cavum conchae inferior at the transition to the external acustic meatus (dorsolateral entrance). Preferably the recording electrode is fixed at this position with an earplug. According to a preferred embodiment of the apparatus the recording electrode is part of or integrated in an earplug of a headset of a stationary or mobile device like music or video
equipment, a (mobile) phone, a PDA, a media player or such. Thus, these devices can be controlled without the need of further hardware, especially without additional electrodes outside the headset. For controlling the devices the same hardware can be used as for hearing music or for a telephone call. Other devices which may be controlled be the apparatus are electric wheelchairs.

For controlling the at least one device the EEG signals detected with the recording electrode are decoded to obtain information about brain activities. By evaluating frequencies in a broad range from theta to gamma oscillatory brain signals recorded with the recording electrode complex cognitive signals are decodable and can be used for controlling the at least one device. In such a way the apparatus works as a brain computer interface (BCI). In a preferred embodiment pattern recognition algorithms, especially a Multivariate Pattern Classifier (MVPC), are used. The pattern recognition comprises in a further preferred embodiment methods of artificial intelligence, like neural networks or support vector machines. In yet another preferred embodiment a frequency transformation, for example (continuous) wavelet, Hilbert, Fourier or other transformation, is applied to single trails of the EEG from at least one of the electrodes, preferably to single trails of each electrode, in order to perform pattern recognition.

According to another aspect of the invention, at least one reference electrode and ground electrode is comprised by the apparatus, where at least one of the at least one reference electrode is arrangeable of the same or opposite side where the at least one recording electrode is attached, and at least one of the at least one ground electrode is arrangeable at the mastoid at the opposite side where a reference electrode is arranged, or at least one of the at least one ground electrode is arrangeable above an ear.

An apparatus for controlling at least one device according to the invention comprises at least one electrode (610) which is arrangeable in the outer ear canal (110), and one data processing unit, the apparatus being configured in such a way that signals of an electroencephalogram (EEG) are detectable by the at least one electrode (610), brain activities are decoded from the signals of the EEG and the at least one device is controlled based on results of the decoding. In a preferred embodiment the apparatus is configured as BCI. As described above, the apparatus may comprise a head set, where the head set may be realized as monoaural or binaural head set. In a further preferred embodiment the apparatus comprises an amplifier nor pre-amplifier, where the (pre-)amplifier preferably is integrated in the head set. For controlling the at least one device the apparatus is communicatively connected to the at least one device. For this purpose, the apparatus provides at least one of a wired and wireless interface. Preferably at least one of the interfaces is integrated in the head set.

A computer program for executing the method according to the invention enables a data processing device, after the program has been loaded into the memory of the data processing device, to carry out a method for controlling at least one device, wherein the data processing device cooperates at least temporarily with at least one recording electrode, and wherein the at least one electrode for detecting signals of an electroencephalogram (EEG) is located in the outer ear canal, brain activities are decoded from the signals of the EEG and the at least one device is controlled based on results of the decoding.

According to a preferred embodiment of the invention, the computer program of the invention has a modular structure, wherein individual modules may be installed on different data processing systems.

Such computer programs may be made available, for example, for downloading in a data or communications network (for a fee or free of charge, unrestricted or password-protected). The provided computer programs can be used by a method, wherein a computer program according to claim 14 is downloaded from an electronic data network, for example from the Internet, to a data processing system connected to the data network.

To carry out the method of the invention, a computer readable storage medium may be employed, on which a program is stored which enables a data processing system, after being loaded into the memory of the data processing system, to carry out a method for controlling at least one device, wherein at least one data processing device cooperates at least temporarily with at least one recording electrode, and wherein the at least one electrode for detecting signals of an electroencephalogram (EEG) is located in the outer ear canal, brain activities are decoded from the signals of the EEG and the at least one device is controlled based on results of the decoding.

According to the invention electrical brain activities are derived from the outer ear canal. For this purpose, a recording electrode is applied to an earplug, for example an earplug of a mobile phone, a PDA or a media player. Using algorithms for pattern recognition the brain activities can be decoded and based on the decoded brain activities applications installed on the mobile device are controlled. The brain activity is recorded as EEG and is subsequently decoded. Thus, devices are controllable solely by intensive thinking.

According to the invention, signals derived from the ear are directly used for decoding brain activities. With the help of the decoded brain activities mental controlling of devices is carried out, i.e. by bio-feedback. A further application area of the invention is cognitive enhancement
via bio-feedback regulation of theta- oscillations (e.g. improvement of memory, see Guderian, Schott, Richardson-Klavehn und Duzel: Medial temporal theta state before an event predicts episodic encoding success in humans. PNAS 2009; 106(13), 5365-5370).

The invention will now be described with reference to several exemplary embodiments illustrated in the drawings, in which.
FIG. 1 is a schematic depiction of the location of a signal electrode at the outer ear canal;
FIG. 2 is a exemplary representation of the theta-power of a subject during a memory task;
FIG. 3 is a exemplary representation of the alpha-power of a subject during a memory task;
FIG. 4 shows examples for indoor and outdoor scenes;
FIG. 5 depicts diagrams showing the accuracy of decoding from EEG activity recorded from a in-ear EEG electrode; and
FIG. 6 provides a schematic drawing of an exemplary embodiment of an in-ear BCI.

In the following an exemplary embodiment of an in-ear BCI 100 is described in greater detail. The exemplary in-ear BCI 100 uses EEG recordings from the external ear canal to decode brain activity in a way that can be used as a brain computer interface using complex cognitive signals.

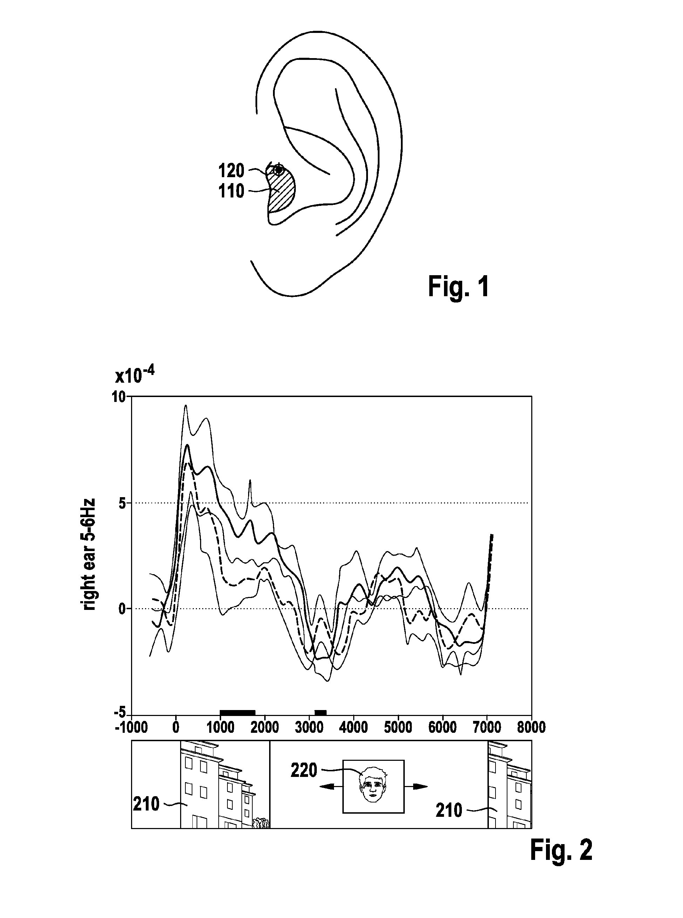

EEG is recorded from the outer ear canal 110. The location 120 of recording is shown in Figure 1. The electrode is positioned at the cavum conchae inferior at the transition to the external acustic meatus (dorsolateral entrance) and is fixated with an earplug.

Recordings are referenced on-line to the ipsi- or contralateral mastoid; ground electrode is the other mastoid. Signals were amplified for instance with a bandwidth of 0.02 - 80 Hz and digitized at a 508.63-Hz sampling rate.

It has been proven that in-ear EEG electrodes can detect oscillatory brain responses to complex cognitive challenges. In-ear EEG recordings are sensitive to brain activity changes in response to complex cognitive demands. This is shown here using a working memory task in which participants have to keep an image 210, 310 depicting a natural scene in mind over a period of 5 seconds. In the middle of the delay a face 220, 320 is presented and participants have to suppress the distracting effect of this face 220, 320. After the 5 seconds they have to decide which of two test images corresponds to the original sample. In half of the trials they are rewarded for correct performance with 0.5 € and in the other half trials there is no reward.

Amplitudes of designated frequencies were assessed separately for each condition. After artifact rejection and band pass filtering (1 - 80 Hz), in an exemplary embodiment of the invention a continuous wavelet transformation was applied to single trails of EEG from each electrode, using 6 cycle Morlet wavelets from 3 to 70 Hz using a logarithmic scaling with 30 steps. In an exemplary embodiment of the invention the 30 wavelet frequencies were 3, 3.34, 3.73, 4.16, 4.63, 5.16, 5.76, 6.42, 7.15, 7.97, 8.89, 9.91, 11.05, 12.31, 13.73, 15.3, 17.06 19.01, 21.19, 23.63, 26.34, 29.36, 32.73, 36.48, 40.67, 45.33, 50.53, 56.33, 62.8, and 70 Hz. For each of the frequencies, the wavelet was convolved with the EEG signal of each trial. The amplitude values were z-normalized for each subject to account for the fact that slower frequencies have higher amplitude values than faster frequencies.

To simplify statistical analysis, the individual-subject amplitude values of designated frequency bands were calculated for each time point. These Frequency bands were theta (5-6 Hz), alpha (10-12 Hz). Amplitude values of the experimental conditions were compared with serial related-measures t-test which were conducted every 17.69 ms. The number of successive significant (at p = 0.005 for each time point) differences between experimental conditions was acquired to be the duration of a full cycle (at a given frequency) divided by 17.69 ms (e.g. for a 10 Hz oscillation, 6 successive data points had to show a significant difference).

### Induced theta oscillations

As can be seen in Figure 2, the onset of the scene image 210 causes a strong increase in theta power which can be measured from the right in-ear electrode. The activity measured at this electrode is also sensitive to the experimental manipulation as to whether performance in a given trial is rewarded or not: the increase of theta power is stronger if performance in the trial is rewarded.

### Alpha desynchronization

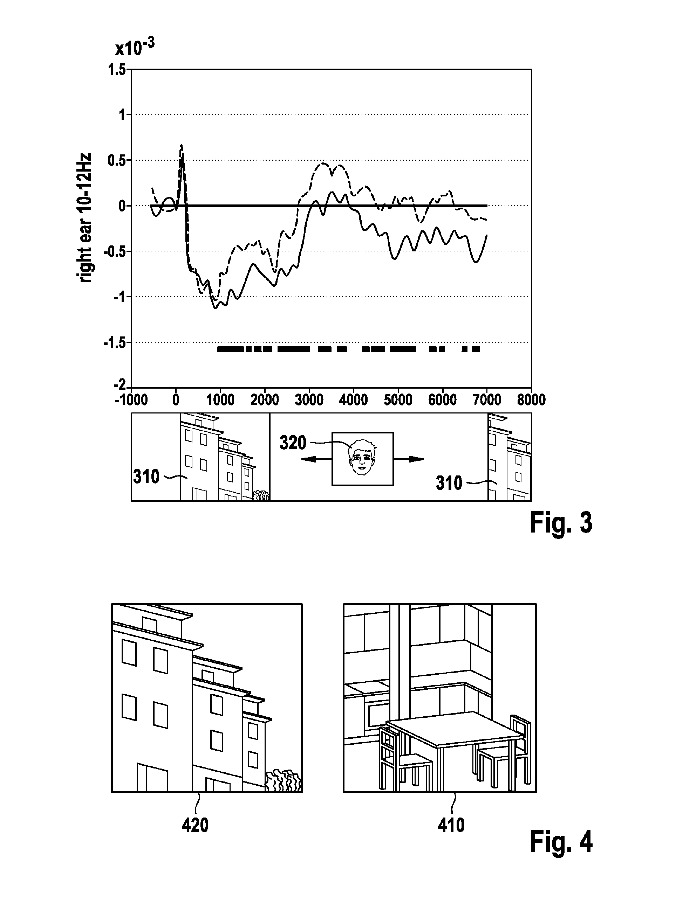

As can be seen in Figure 3, the onset of the scene image 310 causes a strong decrease in alpha power which can be measured from the right in-ear electrode. The activity measured at this electrode is also sensitive to the experimental manipulation as to whether performance in a given trial is rewarded or not: the decrease of alpha power is stronger if performance in the trial is rewarded.

In the following section, it shall be demonstrated that in-ear EEG electrodes can be used to decode brain activity representing complex cognitive information. Individuals were presented with images of single natural scenes that either depicted indoor 410 or outdoor sceneries 420. It had been previously reported (Fuentemilla L., Cashdollar N., Bunzeck N., Penny W., Duzel E.: Theta coupled periodic replay in working memory. Curr Biol, 2010) that it is possible to decode from brain activity whether a participant is currently looking at the image of an indoor 410 or an outdoor scene 420. It is also possible to decode in a memory task whether participants are keeping in mind an indoor 410 or an outdoor scene 420 (in the absence of the image) (Fuentemilla L., Cashdollar N., Bunzeck N., Penny W., Duzel E.: Theta coupled periodic replay in working memory. Curr Biol, 2010). See Figure 4 for examples or an indoor 410 and an outdoor scene 420.

In this example, the decoding is performed with a Multivariate Pattern Classifier (MVPC) algorithm using routines implemented with the Matlab Neural Network Toolbox (Mathworks), but can also be accomplished using other algorithms. In the present example, neural network topology was defined by an input layer, which contained each of the frequency features of the EEG recorded from the in-ear electrode, a hidden layer comprising 20 units, and an output layer, defined by two units, one for each of the category-specific patterns in our study (indoor 410 and outdoor scenes 420). The target patterns were (1 0) for an indoor scene 410 and (0 1) for an outdoor scene 420. Neural network training was always stopped after 20 iterations.

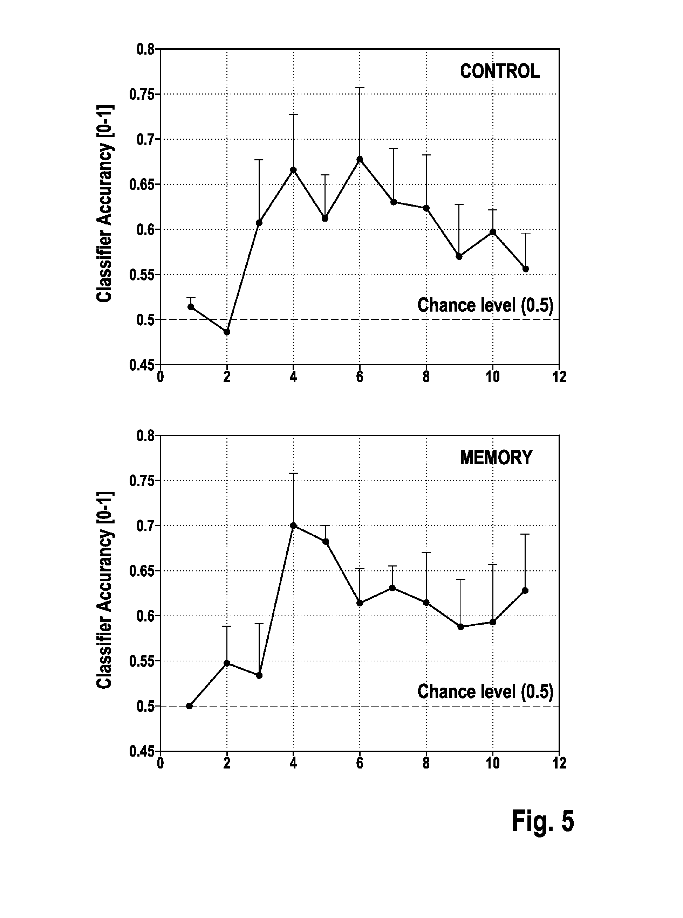

Figure 5 shows the accuracy of decoding from EEG activity recorded with the in-ear EEG electrode whether a subject is currently looking at (control) and memorizing (memory) a indoor 410 or an outdoor scene 420. Hence, in-ear EEG recordings and the frequency decomposition of these EEG recordings are suitable to decode very complex and highly cognitive brain activity contents.

The results summarized above show that in-Ear EEG recordings are suitable for constructing a Brain-Computer Interface (BCI). The in-ear BCI 100 will decode complex mental thoughts and allow individuals to use complex thoughts to generate a variety of cognitive commands to control computers.

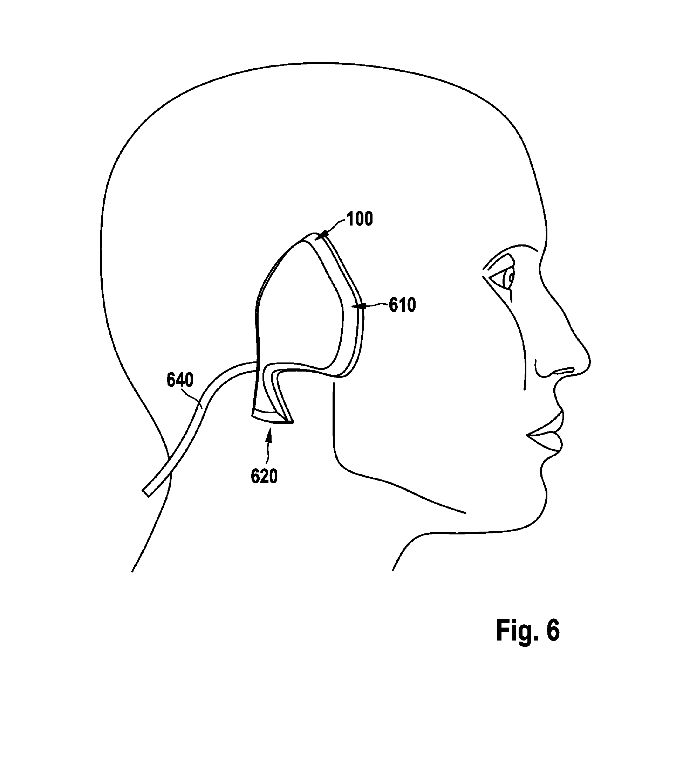

Figure 6 provides a schematic drawing of an exemplary embodiment of an in-ear BCI 100. The exemplary in-ear BCI 100 recording EEG electrode 610 is located inside the outer ear canal and can be attached to an in ear speaker. The exemplary in-ear BCI 100 can be constructed as monoaural or biaural system. The reference electrode 620 on mastoid (same side or other side of the head). The ground electrode 630 above the ear. Connectivity 640 to computer (or phone, ipod or gaming device) can be established via a wire or wireless (e.g. Bluetooth).

There are numerous fields of application for an in-ear BCl according to the invention. In the area of medicine for example, for paralysed individuals, or individuals with language impairment (e.g. after stroke) the in-ear BCI can serve as a portable and easy to use device for communication through complex thoughts and the control of devices (e.g. telephones, electric wheelchairs) through complex mental operations.

In the area of leisure activities, the in-ear BCI can be used in gaming: individuals can learn to control games mentally (e.g. driving a car in a game or using a sword or changing locations or collecting rewards etc.).

The invention is not limited to the exemplary embodiment described above. Instead, additional modified embodiments may be realized through combination and modification of the aforementioned means and features, without going beyond the scope of the invention.

### Reference Signs

- 100: in-ear BCl
- 110: outer ear canal
- 120: location of recording

- 210: image depicting a natural scene
- 220: face

- 310: image depicting a natural scene
- 320: face

- 410: indoor scene
- 420: outdoor scene

- 610: recording EEG electrode
- 620: reference electrode
- 630: ground electrode
- 640: connectivity

## Claims

1. Apparatus for controlling at least one device, the apparatus comprising at least one recording electrode (610) which is arrangeable in the outer ear canal (110), and one data processing unit,
the apparatus being configured in such a way that
signals of an electroencephalogram (EEG) are detectable by the at least one recording electrode (610),
brain activities are decoded from the signals of the EEG and
the at least one device is controlled based on results of the decoding.

2. Apparatus according to claim 1,
**characterized in**
**that** the apparatus is realized as brain computer interface.

3. Apparatus according to claim 1 or 2,
**characterized in**
**that** at least one of the at least one recording electrode (610) is comprised in an earplug.

4. Apparatus according to at least one of the preceding claims,
**characterized in**
**that** the apparatus comprises a head set.

5. Apparatus according to claim 4,
**characterized in**
**that** the head set comprises an amplifier.

6. Apparatus according to at least one of the preceding claims,
**characterized in**
**that** a reference electrode (620) is arrangeable at a mastoid, and that a ground electrode (630) is arrangeable at a mastoid or above an ear.

7. Apparatus according to at least one of the preceding claims,
**characterized in**
**that** the head set is realized as monoaural or binaural head set.

8. Apparatus according to at least one of the preceding claims,
**characterized in**
**that** the apparatus comprises at least one interface for wired and/or wireless communication with the at least one device.

9. Method for controlling at least one device, where
at least one recording electrode (610) for detecting signals of an electroencephalogram (EEG) is located in the outer ear canal (110),
brain activities are decoded from the signals of the EEG and
the at least one device is controlled based on results of the decoding.

10. Method according to claim 9,
**characterized in**
**that** for decoding brain activities at least one of theta, alpha, beta and gamma power which is measured from the at least one recording electrode (610) is evaluated.

11. Method according to claim 9 or 10,
**characterized in**
**that** for decoding brain activities pattern recognition algorithms are performed.

12. Method according to claim 11,
**characterized in**
**that** the pattern recognition algorithms comprise methods of artificial intelligence.

13. Method according to at least one of the claims 9 to 12,
**characterized in**
**that** a frequency transformation is applied to single trails of signals from the at least one recording electrode (610).

14. Computer program which enables a data processing device in cooperation with at least one recording electrode (610), after being loaded into storage means of the data processing device, to carry out a method for controlling at least one device according to at least one of the claims 9 to 13.

15. Computer-readable storage medium, on which a program is stored which enables a data processing device in cooperation with at least one recording electrode (610), after the program is loaded into storage means of the data processing device, to carry out a method for controlling at least one device according to at least one of the claims 9 to 13.
